Europäisches Patentamt

European Patent Office

Office européen des brevets

(11) Publication number: **0 071 185**
**B1**

# EUROPEAN PATENT SPECIFICATION

(45) Date of publication of patent specification: **18.12.85**

(21) Application number: **82106607.3**

(22) Date of filing: **22.07.82**

(51) Int. Cl.⁴: **A 61 B 17/36**

(54) **Laser apparatus.**

(30) Priority: **29.07.81 JP 118681/81**
**19.08.81 JP 122845/81**
**19.08.81 JP 129785/81**
**30.11.81 JP 178211/81**

(43) Date of publication of application:
**09.02.83 Bulletin 83/06**

(45) Publication of the grant of the patent:
**18.12.85 Bulletin 85/51**

(84) Designated Contracting States:
**AT BE CH DE FR GB IT LI NL SE**

(56) References cited:
**US-A-3 710 798**

(73) Proprietor: **OLYMPUS OPTICAL CO., LTD.**
**43-2, 2-chome, Hatagaya Shibuya-ku**
**Tokyo 151 (JP)**

(72) Inventor: **Takano, Akira Olympus Nishihachioji**
**Corporus 121 5-16-1, Sanda-machi**
**Hachioji-shi Tokyo (JP)**
Inventor: **Takei, Toru**
**Olympus Dai 3 Owadaryo 5-19-10, Owada-machi**
**Hachioji-shi Tokyo (JP)**
Inventor: **Toda, Masato**
**Olympus Dai-3-Owadaryo 5-19-10, Owada-**
**Machi**
**Hachioji-shi Tokyo (JP)**
Inventor: **Ichikawa, Saichi**
**2-68-9, Yotsuya**
**Fuchi-shi Tokyo (JP)**

(74) Representative: **Lins, Edgar, Dipl.-Phys. et al**
**Patentanwälte Gramm + Lins Theodor-Heuss-**
**Strasse 2**
**D-3300 Braunschweig (DE)**

## Description

The present invention relates to a laser apparatus and, more particularly, to a laser apparatus used together with an endoscope.

Endoscopic laser apparatuses used together with endoscopes have been developed and are commercially available. According to an endoscopic laser apparatus of this type, a YAG laser oscillator is used to generate an operating laser beam. However, since the YAG laser beam is an invisible light ray, the operator cannot confirm the portion of the body cavity on which the beam is radiated. Undesired portions of the body cavity may therefore be radiated with the operating laser beam. In order to prevent this problem, a visible He-Ne laser beam as a marker laser beam is radiated together with the YAG laser beam as the operating laser beam to perform treatment while confirming the radiated portion of the body cavity with the marker laser beam. However, since the He-Ne laser beam is conventionally radiated continuously on the portion, the color (red) of the He-Ne laser beam cannot be discriminated from that of the radiated portion e.g. the walls of a stomach, so that the operator can hardly confirm the marker beam.

Further, in order to determine whether or not the operating laser beam is emitted, the marker laser beam is conventionally split into two beams by a beam splitter. One marker laser beam is used to confirm radiation of the operating laser beam, while the other marker laser beam is guided to the portion to be treated. Therefore, the amount of the other marker laser beam guided to the portion is small. As a result, it is difficult for the operator to confirm the radiated portion with the marker laser beam.

From US—A—3 710 798 there is known a laser apparatus having

an operating laser oscillator for generating an operating laser beam; a marker laser oscillator for generating a visible marker laser beam; means for guiding the visible marker laser beam coaxially with the operating laser beam to a laser probe of an endoscope; and energizing means for driving said operating laser oscillator in response to the detection. From said US—A—3 710 798 it is also known to provide a chopper for alternately shielding two split marker beams.

It is, therefore, an object of the present invention to provide a laser apparatus with which the operator can easily confirm a portion of a body cavity radiated with an operating laser beam, and which does not allow accidental radiation of the operating laser beam on any portion of the body cavity.

In order to achieve the above object of the present invention, there is provided a laser apparatus comprising:

an operating laser oscillator for generating an operating laser beam; a marker laser oscillator for generating a visible marker laser beam; means for guiding the visible marker laser beam coaxially with the operating laser beam to a laser probe of an endoscope; chopping means for shielding and reflecting the visible marker laser beam from said marker laser oscillator in a predetermined direction at a predetermined frequency; marker laser beam detecting means for detecting the visible marker laser beam shielded and reflected by said chopping means and for generating a detection signal; and energizing means for driving said operating laser oscillator in response to the detection signal from said marker laser beam detecting means.

This invention can be more fully understood from the following detailed description when taken in conjunction with the accompanying drawings, in which:

Fig. 1 is a perspective view of an endoscope system using a laser apparatus according to one embodiment of the present invention;

Fig. 2 is a schematic view of the endoscope system including a block diagram of the laser apparatus shown in Fig. 1;

Fig. 3 is a plan view of a chopper shown in Fig. 2;

Fig. 4 is a sectional view of the chopper shown in Fig. 3;

Fig. 5 is a timing chart of signals appearing in the circuit of the laser apparatus in Fig. 2;

Fig. 6 is a schematic view of an endoscope system using a laser apparatus according to another embodiment of the present invention;

Fig. 7 is a side view of a chopper used in the laser apparatus shown in Fig. 6; and

Fig. 8 is a sectional view of the chopper shown in Fig. 7.

Fig. 9 is a schematic view of an endoscope system using a laser apparatus according still another embodiment of the present invention;

Fig. 10 shows a timing chart for explaining the mode of operation of the laser apparatus shown in Fig. 9;

Fig. 11 is a schematic view of an endoscope system using a laser apparatus according to still another embodiment of the present invention; and

Figs. 12 to 14 respectively show modifications of the chopper used in the laser apparatus according to the present invention.

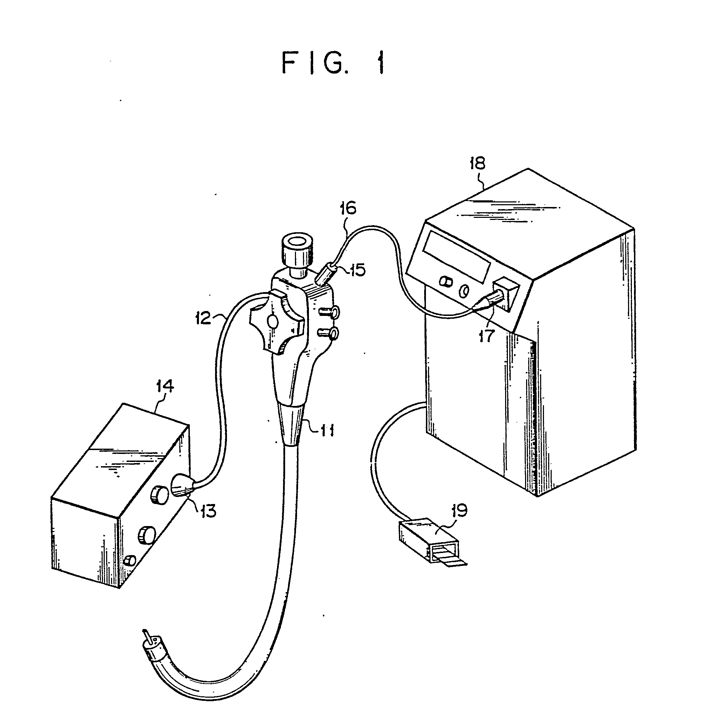

According to the endoscope system shown in Fig. 1, a connector 13 mounted at the distal end of a universal cord 12 of an endoscope 11 is connected to a light supply unit 14. A laser probe 16 is inserted into a forceps channel from a forceps channel inlet 15 of the endoscope 11. A connector 17 of the laser probe 16 is coupled to a laser apparatus 18.

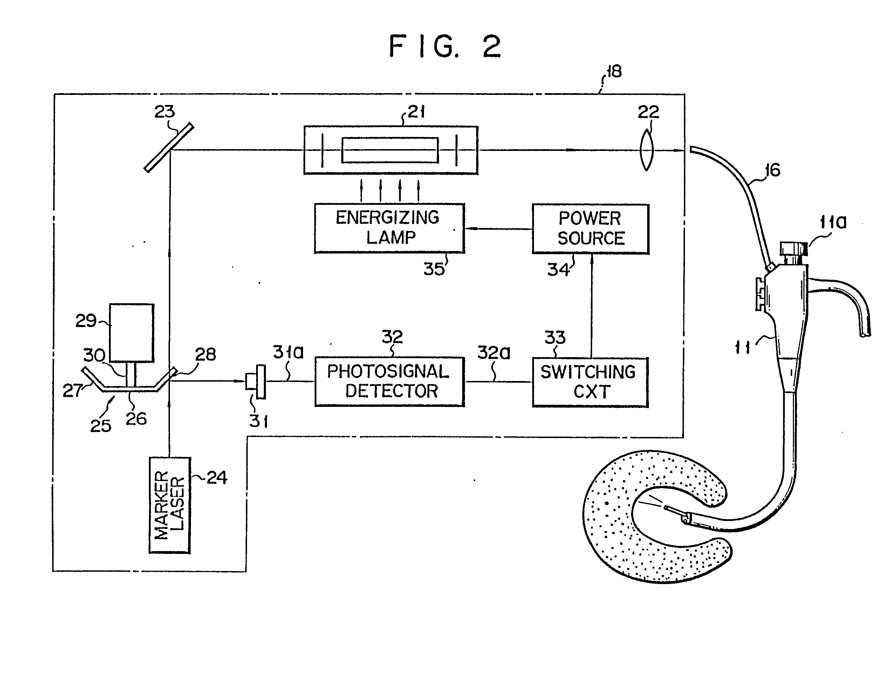

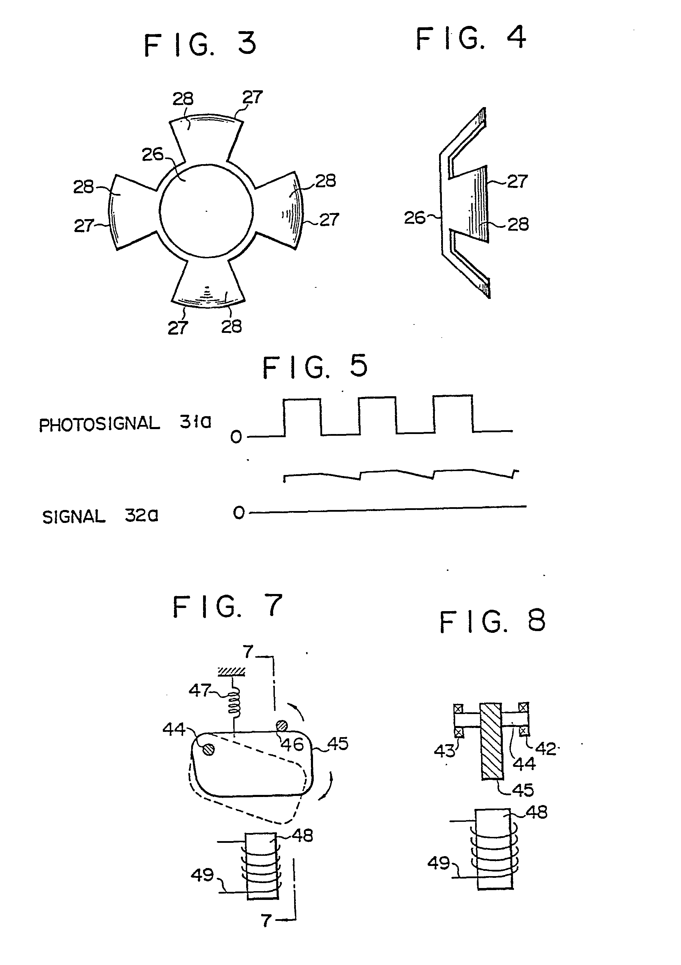

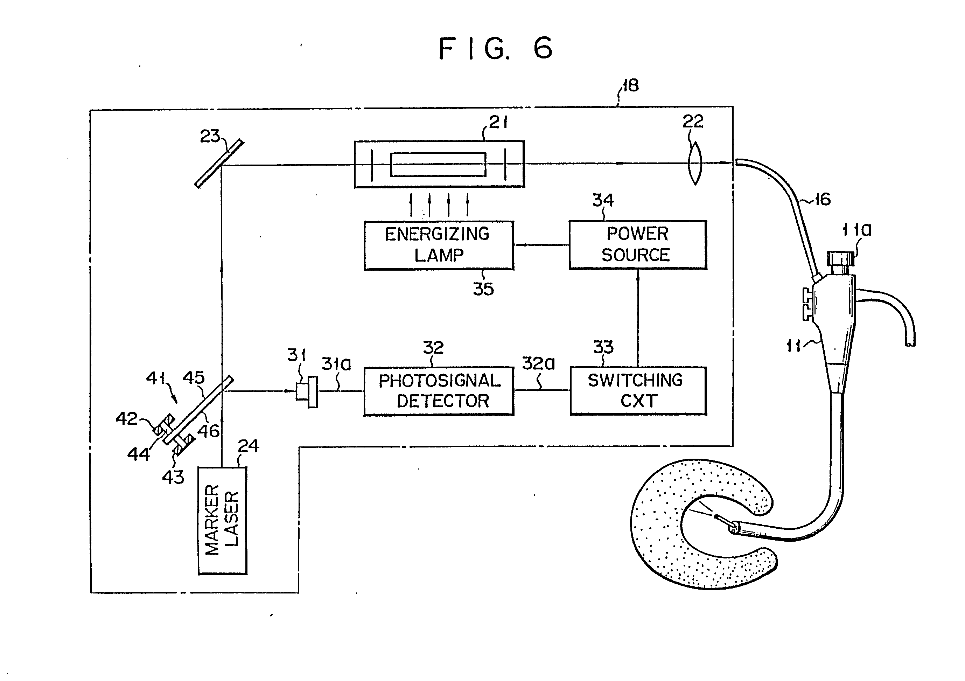

An operating laser oscillator 21 such as a YAG laser oscillator is arranged in the laser apparatus 18, as shown in Fig. 2. A focusing lens 22 is arranged on the optical axis and at the output side of the operating laser oscillator 21. The focusing lens 22 focuses the laser beam on the laser probe 16. A reflecting mirror 23 is disposed on the optical axis as tilted at, for example, 45° with respect thereto and is located at the side of the operating laser oscillator 21 opposite to the output side described above. A marker laser oscillator 24 such as a helium-neon (He-Ne) laser oscillator is arranged perpendicular to the optical axis of the operating laser oscillator 21 and opposes the reflecting mirror 23. A chopper 25 is interposed between the reflecting mirror 23 and the marker laser oscillator 24. The chopper 25 which is a substantially truncated conical rotary body has a base plate 26 and reflecting plates 27, as shown in Figs. 3 and 4. The reflecting plates comprise vanes which are integrally formed at equal intervals on the periphery of the base plate 26 and which extend therefrom at, for example, 45°. A reflecting surface 28 is formed on the outer surface of each reflecting plate 27. A rotating shaft 30 of a motor 29 which is parallel to the optical axis of the marker laser oscillator 24 is rotatably mounted at the center of the base plate 26.

A photosensor 31 opposes the reflecting surface 28 of the chopper 25 and is perpendicular to the optical axis of the marker laser oscillator 24. The output terminal of the photosensor 31 is connected to the input terminal of a photosignal detector 32. The photosignal detector 32 comprises an integrator circuit for integrating an output from the photosensor 31. The output terminal of the photosignal detector 32 is connected to the control input terminal of a switching circuit 33. The control output terminal of the switching circuit 33 is connected to the on-off terminal of a power source 34. The output terminal of the power source 34 is connected to an energizing lamp 35 arranged in the vicinity of the operating laser oscillator 21.

The mode of operation of the laser apparatus with the above arrangement will be described. When the operator turns on a power switch of the light supply unit 14, the observation light is guided through a light guide (not shown) of the endoscope 18 and illuminates the body cavity. When the operator then turns on a main switch of the laser apparatus 18, the motor 29 is driven to rotate the chopper 25. The marker laser oscillator 24 then oscillates to generate a marker laser beam. The marker laser beam from the marker laser oscillator 24 alternately becomes incident on the reflecting mirror 23 and is reflected by the reflecting surface 28 of the chopper 25 in the direction perpendicular to the incident direction, according to the rotation of the chopper 5. The marker laser beam incident on the reflecting mirror 23 is focused by the focusing lens 22 through the operating laser oscillator 21 and is incident on the laser probe 16 of the endoscope 11. The marker laser beam emerging from the laser probe 16 is radiated on the wall of the body cavity. The operator can observe the radiated wall portion through an eyepiece 11a of the endoscope 11. The operator observes the flashing marker laser beam. The flashing frequency is determined by the rotational speed of the chopper 25 to be from several Hz to several tens of Hz, which allows proper observation with the naked eye. When the operator confirms the flashing of the marker laser beam and hence the portion of the body cavity to be radiated, he depresses a foot switch 19. The switching circuit 33 is then actuated. In this condition, the photosensor 31 receives the marker laser beam intermittently reflected by the reflecting surfaces 28 of the chopper 25 and produces a photosignal 31a with a waveform shown in Fig. 5. The photosignal 31a from the photosensor 31 is then converted to an integration signal 32a by the photosignal detector 32. When the integration signal 32a is supplied to the switching circuit 33, the switching circuit 33 causes the power source 34 to be ON. The power source 34 supplies a lighting current to the energizing lamp 35 to turn it on. The pumping light from the energizing lamp 35 excites the operating laser oscillator 21 to generate the operating laser beam. The operating laser beam is coaxially focused together with the marker laser beam by the focusing lens 22 onto the laser probe 16. The operating laser beam is radiated on the predetermined portion of the body cavity for treatment through the laser probe 16. In this case, since the marker laser beam flashes and is superposed on the operating laser beam, the operator can confirm the portion to be radiated with the operating laser beam and perform the proper treatment. When the operator releases the foot switch 19, the radiation of the operating laser beam is stopped. Only the marker laser beam flashes on the radiated portion.

As described above, since the marker laser beam flashes, the portion radiated by the operating laser beam can be easily confirmed even if the color of the radiated portion is similar to that of the marker laser beam. Further, only when the marker laser beam is emitted, the operating laser oscillator is driven. Therefore, an undesired portion of the body cavity will not be accidentally radiated by the operating laser beam, thus achieving safe treatment.

According to the laser apparatus shown in Fig. 6, a chopper 41 has a reflecting plate 45 which is free to pivot and which is mounted on a shaft 44 rotatably mounted on bearings 42 and 43. The reflecting plate 45 is inclined at, for example, 45° with respect to the marker laser oscillator 24 and the photosensor 31. A spring 47 is mounted on the upper portion of the reflecting plate 45, as shown in Fig. 7. The reflecting plate 45 abuts against a stopper 46 by the urging force of the spring 47. In this condition, the marker laser beam is reflected toward the photosensor 31. An electromagnet 48 is disposed below the reflecting plate 45, as shown in Figs. 7 and 8. When an energizing current is supplied to a coil 49 of the electromagnet 48, the reflecting plate 45 is attracted downward by the magnetic force of the electromagnet 48 against the urging force of the spring 47. Thus, the marker laser beam is incident on the reflecting mirror 23. When a pulse current of several Hz to several tens of Hz is supplied to the coil 49, the reflecting plate 45 pivots. The marker laser beam flashes and is incident on the laser probe 16 and the photosensor 31. Therefore, in the same manner as in the first embodiment, the radiated portion can be properly confirmed. Any undesired portion of the body cavity will not be accidentally radiated by the operating laser beam without confirmation.

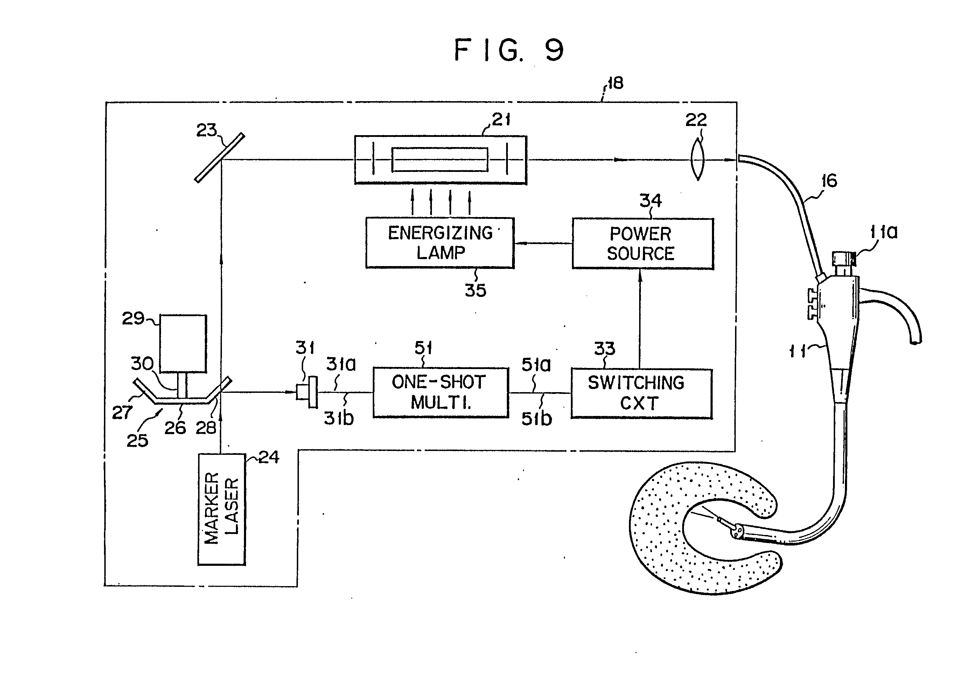

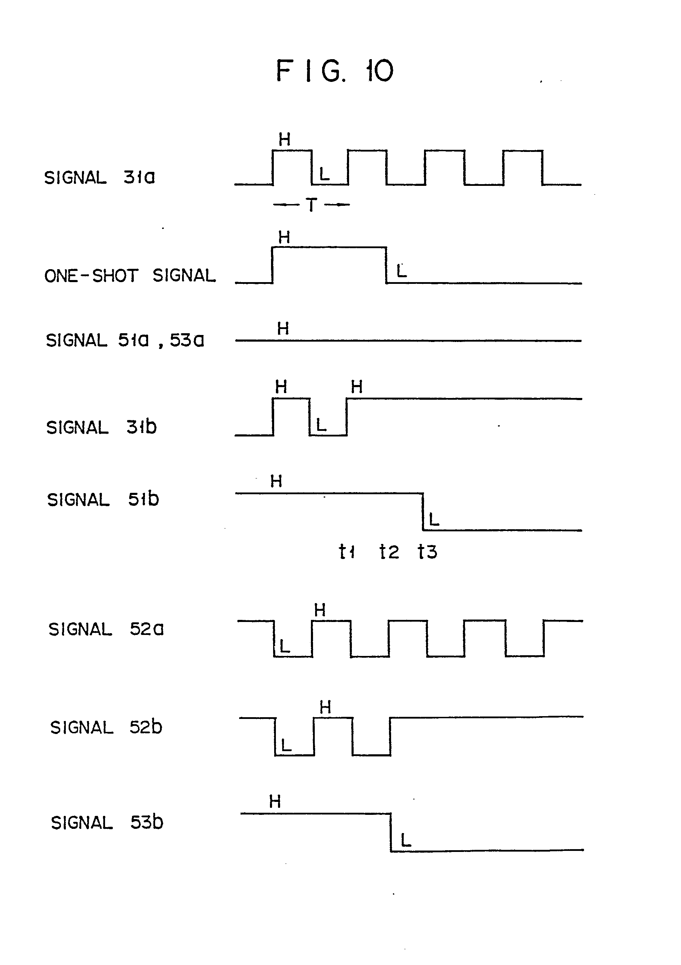

A laser apparatus according to a third embodiment of the present invention will be described with reference to Fig. 9. The same reference numerals used in the previous embodiments denote the same or similar parts in Fig. 9, and a detailed description thereof will be omitted. In the third embodiment, a one-shot multivibrator 51 is used as the photosignal detector. The one-shot multivibrator 51 has a time constant larger than the period T of the photosignal 31a generated by the photosensor 31 when the chopper 25 is rotated at a predetermined speed. The one-shot multivibrator 51 produces a signal 51a of a waveform shown in Fig. 10 in response to the leading edge of the first pulse of the photosignal 31a. While the photosensor 31 produces the signal 31a of the period T, the one-shot multivibrator 51 produces the signal 51a of high level H. The switching circuit 33 drives the power source 34 in response to the signal 51a. The power source 34 then turns on the energizing lamp 35 amd the operating laser oscillator 21 is driven to generate the operating laser beam. The marker laser beam passing though a space between the reflecting plates 27 of the chopper 25 becomes coaxial with the operating laser beam. Thus, the marker laser beam guided through the laser probe 16 of the endoscope 11 intermittently illuminates the portion to be treated, indicating the radiation position of the operating laser beam.

If the chopper 25 is broken and stops rotating while the marker laser beam is shielded by the reflecting plate 27 thereof, the marker laser beam is continuously reflected by the reflecting plate 27 and is continuously incident on the photosensor 31. The photosensor 31 then produces the signal 31b. The signal 31b is set to high level H after time t1. The one-shot multivibrator 51 is not retriggered at time t3, so that it produces a signal 51b which goes low at time t3. The signal 51b of low level L resets the switching circuit 33 to turn off the power source 34, whereby the operating laser oscillator 21 stops oscillation. If the marker laser beam is shielded by the chopper 25 and is not incident on the laser probe 16 so that the radiation position of the operating laser beam can not be confirmed by the marker laser beam, the operating laser beam is not be generated. Therefore, the operating laser beam is not be radiated on any portion of the body cavity when the operator cannot confirm the portion with the marker laser beam.

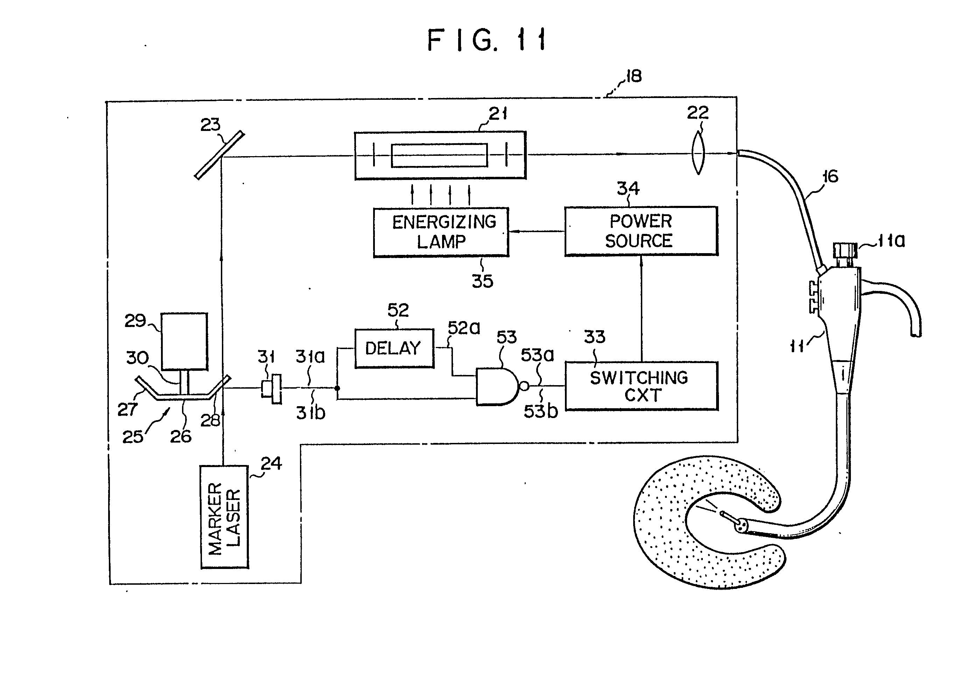

In a fourth embodiment shown in Fig. 11, a delay circuit 52 and a NAND gate 53 are used as the photosignal detector. The input terminal of the delay circuit 52 is connected to the output terminal of the photosensor 31 and the output terminal thereof is connected to one input terminal of the NAND gate 53. The other input terminal of the NAND gate 53 is connected directly to the output terminal of the photosensor 31. According to this embodiment, the delay circuit 52 delays the signal 31a from the photosensor 31 by T/2 and produces a signal 52a to one input terminal of the NAND gate 53. The signal 31a from the photosensor 31 is directly supplied to the other input terminal of the NAND gate 53. The NAND gate 53 produces a signal 53a of high level. In this condition, the operating laser oscillator 21 starts oscillation to generate the operating laser beam. Thus, the operating laser beam together with the marker laser beam is incident on the laser probe 19.

When the chopper 25 is broken and the marker laser beam is shielded by the reflecting plate 27, the signal 31b of high level is produced by the photosensor 31. As a result, the signal 52a is produced by the delay circuit 52 which is supplied together with the signal 31b to the NAND gate 53. A signal 53b which goes low at time t2 is produced by the NAND gate 53. The switching circuit 33 is then reset, and the operating laser oscillator 21 stops oscillation. In the same manner as in the third embodiment of Fig. 9, when the marker laser beam is shielded over a predetermined period of time, the operating laser beam is stopped.

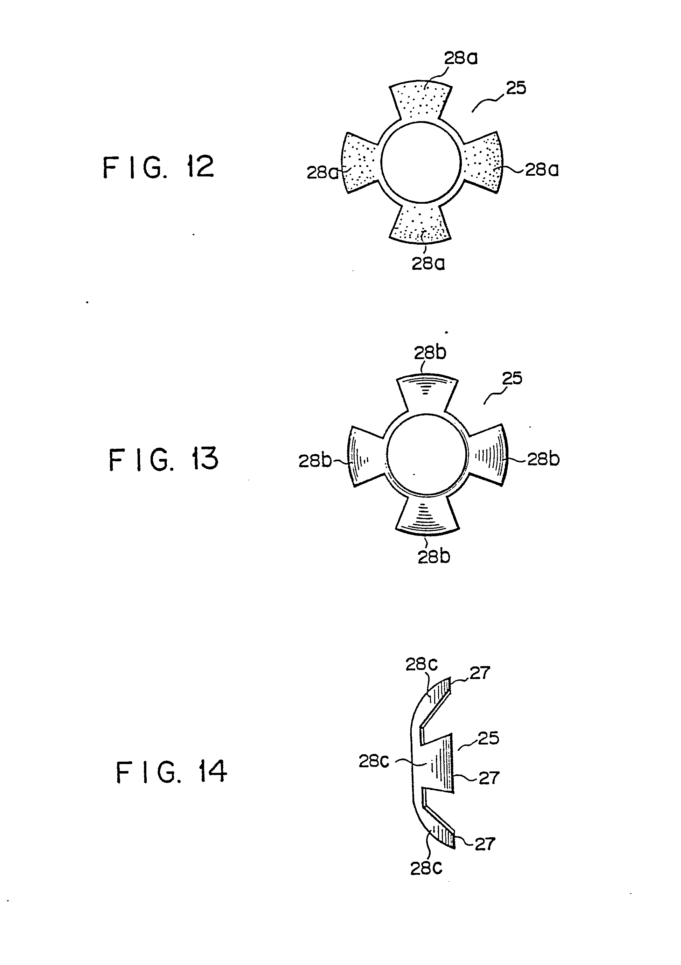

In the laser apparatus described above, if the reflecting surface 28 comprises a simple glossy surface, the marker laser beam is regularly reflected by the reflecting surface 28. If the positional relationship between the reflecting surface 28 and the photosensor 31 is changed, the reflected beam may not be detected by the photosensor 31. In order to avoid this, the reflecting surface 28a of the chopper 25 is finished with an aventurine lacquer as shown in Fig. 12. Alternatively, a number of grooves are formed along the circumferential direction of the reflecting surface 28b to irregularly reflect the beam, as shown in Fig. 13. The chopper 25 may be of a conical shape, as shown in Fig. 14. In this case, a reflecting surface 28c is a curved surface, so that the marker laser beam which is incident on the surface 28a is diffuse-reflected. In this manner, since the reflecting surface of the chopper 25 comprises an irregularly reflecting surface or a diffuse-reflecting surface, the reflected marker laser beam is properly detected by the photosensor.

In summary, according to the present invention, since the marker laser beam is selectively and intermittently guided to the laser probe of the endoscope and to the operating laser oscillator control section by the chopper, the operator can properly confirm the portion to be radiated by the flashing of the marker laser beam. Further, only when the marker laser beam is generated, the operating laser beam is generated, thus achieving safe treatment of the desired portion of the body cavity with a laser beam.

**Claims**

1. A laser apparatus comprising:
an operating laser oscillator (21) for generating an operating laser beam;

a marker laser oscillator (24) for generating a visible laser beam;

means (23) for guiding the visible marker laser beam coaxially with the operating laser beam to a laser probe of an endoscope;

chopping means (25, 41) for shielding and reflecting the visible marker laser beam from said marker laser oscillator (24) in a predetermined direction at a predetermined frequency;

marker laser beam detecting means (31, 32) for detecting the visible marker laser beam shielded and reflected by said chopping means (25, 41) and for generating a detection signal; and

energizing means (34, 35) for driving said operating laser oscillator (21) in response to the detection signal from said marker laser beam detecting means.

2. An apparatus according to claim 1, wherein said marker-laser beam chopping means comprises a chopper (25) which is interposed between said marker laser oscillator (24) and said guiding means (23), which rotates at a predetermined speed, and which has a plurality of vanes (27) each having a reflecting surface (28) inclined with respect to a travelling direction of the visible marker laser beam (24).

3. An apparatus according to claim 1, wherein said marker-laser beam chopping means comprises a chopper plate (45) which is interposed between said marker laser oscillator (24) and said guiding means (23), which has a reflecting surface inclined with respect to the travelling direction of the visible marker laser beam, and which shields the visible marker laser beams at a predetermined frequency by pivoting at the predetermined frequency.

4. An apparatus according to claim 1, 2 or 3, wherein the predetermined frequency is in a range of several Hz to several tens of Hz.

5. An apparatus according to claim 1, 2 or 3, wherein said marker laser beam detecting means comprises a photosensor (31) receiving the visible marker laser beam reflected by said chopping means to convert the visible laser beam into the photoelectric signal of the predetermined frequency, and a one-shot multivibrator (51) which is connected to said photosensor (31), which has a time constant larger than a period of the photoelectric signal, and which is triggered at the predetermined frequency of the photoelectric signal.

6. An apparatus according to claim 1, 2, or 3, wherein said marker laser beam detecting means comprises a photosensor (31) receiving the visible marker laser beam reflected by said chopping means to convert the visible beam into the photoelectric signal of the predetermined frequency, a delay circuit (52) connected to said photosensor (31) to delay the photoelectric signal by 1/2 a period thereof, and means (53) for producing a NAND signal between an output signal from said delay circuit and the photoelectric signal from said photosensor (31).

7. An apparatus according to any of claim 1 to 6, wherein said energizing means comprises an energizing lamp (35) disposed near said operating laser oscillator (21) and means (34) for turning on said energizing lamp (35) in response to the detection signal from said marker laser beam detecting means.

8. An apparatus according to any one of claims 1 to 7, wherein said operating laser oscillator (21) comprises a YAG laser oscillator.

9. An apparatus according to any one of claims 1 to 8, wherein said marker laser oscillator (24) comprises a helium-neon laser oscillator.

10. An apparatus according to any one of claims 1 to 9, wherein the predetermined frequency is in a range of several Hz to twenty Hz.

11. An apparatus according to any one of claims 2 to 10, wherein said reflecting surface of said chopping means comprises an irregular reflecting surface (28a, 28b).

12. An apparatus according to claim 11, wherein said reflecting surface comprises an aventurine lacquer finished surface (28a).

13. An apparatus according to claim 11, wherein said irregular reflecting surface comprises a reflecting surface (28b) with a number of grooves extending along a circumferential direction thereof.

14. An apparatus according to any one of claims 2 to 10, wherein said reflecting surface comprises a diffuse-reflecting surface (28c).

15. An apparatus according to claim 14, wherein said diffuse-reflecting surface comprises a curved reflecting surface (28c).

**Patentansprüche**

1. Lasergerät mit
einem Behandlungslaseroszillator (21) zur Erzeugung eines Behandlungslaserstrahls,
einem Markierungslaseroszillator (24) zur Erzeugung eines sichtbaren Markierungslaserstrahls,
einer Einrichtung (23) zur Führung des sichtbaren Markierungslaserstrahls kooaxial mit dem Behandlungslaserstrahl auf eine Lasersonde eines Endoskops,
einer Unterbrechereinrichtung (25, 41) zum Abschirmen und Reflektion des sichtbaren Markierungslaserstrahls des Markierungslaseroszillators (24) in einer vorbestimmten Richtung mit einer vorbestimmten Frequenz,
einer Markierungslaserstrahl-Detektionseinrichtung (31, 32) zur Detektion des von der Unterbrechereinrichtung (25, 41) abgeschirmten und reflektierten sichtbaren Markierungslaserstrahls und zur Erzeugung eines Detektionssignals und
einer Erregereinrichtung (34, 35) zum Steuern des Behandlungslaseroszillators (21) in Abhängigkeit von dem Detektionssignal der Markierungslaserstrahl-Detektionseinrichtung.

2. Lasergerät nach Anspruch 1, in dem die Unterbrechereinrichtung für den Markierungslaserstrahl einen Unterbrecher (25) aufweist, der zwischen dem Markierungslaseroszillator (24) und der Führungseinrichtung (23) angeordnet ist, mit einer vorbestimmten Geschwindigkeit rotiert und der eine Mehrzahl von Flügeln (27) aufweist, die jeder eine zur Fortpflanzungsrichtung des sichtbaren Markierungslaserstrahls geneigte reflektierende Oberfläche (28) aufweisen.

3. Lasergerät nach Anspruch 1, in dem die Unterbrechereinrichtung für den Laserstrahl eine Unterbrecherplatte (45) aufweist, die zwischen dem Markierungslaseroszillator (24) und der Führungseinrichtung (23) angeordnet ist, eine zur Fortpflanzungsrichtung des sichtbaren Markierungslaserstrahls geneigte reflektierende Oberfläche aufweist und die sichtbaren Markierungslaserstrahlen mit einer vorbestimmten Frequenz durch eine Drehbewegung mit der vorbestimmten Frequenz abschirmt.

4. Lasergerät nach Anspruch 1, 2 oder 3, bei dem sich die vorbestimmte Frequenz im Bereich von einigen Hz bis einigen 10 Hz bewegt.

5. Lasergerät nach Anspruch 1, 2 oder 3, bei dem die Markierungslaserstrahl-Detektionseinrichtung einen Fotosensor (31), der den von der Unterbrechungseinrichtung reflektierten sichtbaren Markierungslaserstrahl empfängt und den sichtbaren Laserstrahl in ein fotoelektrisches Signal der vorbestimmten Frequenz wandelt, und einen Monovibrator (51) aufweist, der mit dem Fotosensor (31) verbunden ist, eine Zeitkonstante hat, die größer als die Periode des fotoelektrischen Signals ist und der mit der vorbestimmten Frequenz des fotoelektrischen Signals getriggert wird.

6. Lasergerät nach Anspruch 1, 2 oder 3, bei dem die Markierungslaserstrahl-Detektionseinrichtung einen Fotosensor (31), der den von der Unterbrechereinrichtung reflektierten sichtbaren Markierungslaserstrahl empfängt und den sichtbaren Laserstrahl in ein fotoelektrisches Signal mit der vorbestimmten Frequenz wandelt, eine Verzögerungsschaltung (52), die an den Fotosensor (31) angeschlossen ist, um das fotoelektrische Signal um eine halbe Periode dieses Signals zu verzögern, und eine Einrichtung (53) zur Erzeugung eines NAND-Signals zwischen einem Ausgangssignal der Verzögerungsschaltung dem fotoelektrischen Signal von dem Fotosensor (31) aufweist.

7. Lasergerät nach einem der Ansprüche 1 bis 6, bei dem die Erregereinrichtung eine nahe des Behandlungslaseroszillators (21) angeordnete Erregerlampe (35) und eine Einrichtung (34) zum Einschalten der Erregerlampe (35) in Abhängigkeit von dem Detektionssignal der Markierungslaserstrahl-Detektionseinrichtung aufweist.

8. Lasergerät nach einem der Ansprüche 1 bis 7, bei dem der Behandlungslaseroszillator (21) einen YAG-Laseroszillator umfaßt.

9. Lasergerät nach einem der Ansprüche 1 bis 8, bei dem der Markierungslaseroszillator (24) einen Helium-Neon-Laseroszillator umfaßt.

10. Lasergerät nach einem der Ansprüche 1 bis 9, bei dem die vorbestimmte Frequenz im Bereich von einigen Hz bis 20 Hz liegt.

11. Lasergerät nach einem der Ansprüche 2 bis 10, bei dem die reflektierende Oberfläche der Unterbrechereinrichtung eine unregelmäßig reflektierende Oberfläche (28a, 28b) aufweist.

12. Lasergerät nach Anspruch 11, bei dem die reflektierende Oberfläche eine mit Aventurinlack behandelte Oberfläche (28a) aufweist.

13. Lasergerät nach Anspruch 11, bei dem die unregelmäßig reflektierende Oberfläche eine mit Rillen versehene reflektierende Oberfläche (28b) aufweist, die sich entlang deren Umfangfläche erstrecken.

14. Lasergerät nach einem der Ansprüche 2 bis 10, bei dem die reflektierende Oberfläche eine diffus reflektierende Oberfläche (28c) aufweist.

15. Lasergerät nach Anspruch 14, bei dem die diffus reflektierende Oberfläche eine gekrümmte reflektierende Oberfläche (28c) aufweist.

**Revendications**

1. Appareil à laser comprenant: un oscillateur 21 à laser de travail pour créer un rayon laser de travail; un oscillateur (24) à laser de repère pour créer un rayon laser visible de repère; un moyen (23) pour guider le rayon laser visible de repère coaxialement au rayon laser de travail vers une sonde à laser d'un endoscope; un moyen (25, 41) d'interruption pour intercepter et réfléchir le rayon laser visible de repère envoyé par l'oscillateur (24) à laser de repère dans une direction prédéterminée et à une fréquence prédéterminée; un moyen (31, 32) de détection du rayon laser de repère pour détecter le rayon laser visible de repère intercepté et réfléchi par le moyen (25, 41) d'interruption et pour créer un signal de détection; et un moyen (34, 35) de stimulation pour déclencher l'oscillateur (21) à laser de travail en réponse au signal de détection émis par le moyen (31, 32) de détection du rayon laser de repère.

2. Appareil suivant la revendication 1, dans lequel le moyen d'interruption du rayon laser de repère comprend un interrupteur (25) de rayon qui est interposé entre l'oscillateur (24) à laser de repère et le moyen 23 de guidage, qui tourne à une vitesse prédéterminée et qui comporte une pluralité d'ailettes (27) ayant chacune une face réfléchissante (28) inclinée par rapport à la direction de propagation du rayon laser visible de repère.

3. Appareil suivant la revendication 1, dans lequel le moyen d'interruption du rayon laser de repère comporte une plaquette (45) d'interruption qui est interposée entre l'oscillateur (24) à laser de repère at le moyen (23) de guidage, qui présente une face réfléchissante inclinée par rapport à la direction de propagation du rayon laser visible de repère, et qui intercepte les rayons laser visibles de repère à une fréquence prédéterminée par pivotement à une fréquence prédéterminée.

4. Appareil suivant l'une des revendications 1, 2 ou 3, dans lequel la fréquence prédéterminée est de l'ordre de plusieurs Hz à plusieurs dizaines de Hz.

5. Appareil suivant l'une des revendications 1, 2 ou 3 dans lequel le moyen de détection du rayon laser de repère comprend un photodétecteur (31) recevant le rayon laser visible de repère réfléchi par le moyen d'interruption pour convertir ce rayon laser visible en un signal photo-électrique de fréquence prédéterminée, et un multivibrateur monostable (51) qui est connecté au photodétecteur (31), qui a une constante de temps supérieure à la période du signal photoélectrique, et qui est déclenché à la fréquence prédéterminée du signal photoélectrique.

6. Appareil suivant l'une des revendications 1, 2 ou 3, dans lequel le moyen de détection du rayon laser de repère comprend un photodétecteur (31) recevant le rayon laser visible de repère réfléchi par le moyen d'interruption pour convertir ce rayon laser visible en un signal photo-électrique de fréquence prédéterminée, un circuit (52) à retard relié au photodétecteur (31) pour retarder de 1/2 période le signal photo-électrique, et un moyen (53) pour créer un signal NON-ET entre un signal de sortie du circuit à retard et le signal photo-électrique émis par le photodétecteur (31).

7. Appareil suivant l'une quelconque des revendications 1 à 6, dans lequel le moyen de stimulation comprend une lampe (35) de stimulation disposée près de l'oscillateur (21) à laser de travail et un moyen (34) pour allumer cette lampe (35) de stimulation en réponse au signal de détection venant du moyen de détection du rayon laser de repère.

8. Appareil suivant l'une quelconque des revendications 1 à 7, dans lequel l'oscillateur (21) à laser de travail est constitué d'un oscillateur à laser à YAG.

9. Appareil suivant l'une quelconque des revendications 1 à 8, dans lequel l'oscillateur (24) à laser de repère comprend un oscillateur à laser à hélium-néon.

10. Appareil suivant l'une quelconque des revendications 1 à 9, dans lequel la fréquence prédéterminée est de l'ordre de plusieurs Hz à vingt Hz.

11. Appareil suivant l'une quelconque des revendications 2 à 10, dans lequel la face réfléchissante du moyen d'interruption comprend une surface réfléchissante irrégulière (28a, 28b).

12. Appareil suivant la revendication 11, dans lequel la face réfléchissante comprend une surface (28a) peinte à l'aide d'une laque à l'aventurine.

13. Appareil suivant la revendication 11, dans lequel la face irrégulière réfléchissante comprend une surface réfléchissante (28b) avec un certain nombre de stries formées concentriquement à la circonférence de cette surface.

14. Appareil suivant l'une quelconque des revendications 2 à 10, dans lequel la face réfléchissante comprend une surface (28c) à réflexion diffuse.

15. Appareil suivant la revendication 14, dans lequel cette surface à réflexion diffuse comprend une surface réfléchissante courbe (28c).

# F I G. 1

## F I G. 2

# FIG. 3

# FIG. 4

# FIG. 5

PHOTOSIGNAL  31a

SIGNAL  32a

# FIG. 7

# FIG. 8

# FIG. 6

# FIG. 9

# F I G. 10

SIGNAL 31a

ONE-SHOT SIGNAL

SIGNAL 51a , 53a

SIGNAL 31b

SIGNAL 51b

SIGNAL 52a

SIGNAL 52b

SIGNAL 53b

# FIG. 11

0 071 185

F I G. 12

F I G. 13

F I G. 14